Europäisches Patentamt
European Patent Office
Office européen des brevets

(19)

(11) Publication number: **0 192 606**
**A2**

(12) # EUROPEAN PATENT APPLICATION

(21) Application number: 86810076.9

(22) Date of filing: 11.02.86

(51) Int. Cl.[4]: A 01 N 43/56
C 07 D 403/12, C 07 D 239/48

(30) Priority: 22.02.85 GB 8504609

(43) Date of publication of application:
27.08.86 Bulletin 86/35

(84) Designated Contracting States:
AT BE CH DE FR GB IT LI NL

(71) Applicant: SANDOZ AG
Lichtstrasse 35
CH-4002 Basel(CH)

(84) Designated Contracting States:
BE CH FR GB IT LI NL

(71) Applicant: SANDOZ-PATENT-GMBH
Humboldtstrasse 3
D-7850 Lörrach(DE)

(84) Designated Contracting States:
DE

(71) Applicant: SANDOZ-ERFINDUNGEN
Verwaltungsgesellschaft m.b.H.
Brunner Strasse 59
A-1235 Wien(AT)

(84) Designated Contracting States:
AT

(72) Inventor: Kuhnen, Fred
Reblistrasse 20
D-7858 Weil am Rhein(DE)

(72) Inventor: Milzner, Karlheinz
Kannenfeldstrasse 27
CH-4056 Basel(CH)

(72) Inventor: Seckinger, Karl
Bergstrasse 19
D-7831 Riegel(DE)

(54) **Herbicidal chloroacetamides.**

(57) The invention provides a method of controlling perennial weeds with the aid of N-(pyrazolylalkyl)-N-(5-pyrimidinyl)-chloroacetamide compounds of formula I

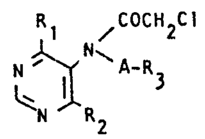

I

wherein

$R_1$ is a N,N-disubstituted amino group,
$R_2$ is halogen, $C_{1-4}$alkyl, or an ether or thio ether group,
$R_3$ is 1-pyrazolyl or 3,5-dimethyl-1-pyrazolyl, and
A is $CH_2$ or $CH(CH_3)$,

in free base form or in agriculturally acceptable acid addition salt form, herbicidal compositions comprising such compounds and certain novel compounds belonging to the general class of compounds of formula I and which are particularly suitable for use in the method of the invention.

EP 0 192 606 A2

0192606

Case 130-3978

# HERBICIDAL CHLOROACETAMIDES

The present invention relates to the use of N-(5-pyrimidinyl)-chloroacetamides in combatting perennial weeds.

U.K. Patent Spec. No. 2 127 404 discloses N-substituted -N'-(5-pyrimidinyl)-chloroacetamides and the use of such compounds in combatting dicotyledoneous and grassy weeds.

Though these and many other herbicides may damage certain upperground parts of perennial weeds, there efficacy as a herbicide is usually limited in that they do not suppress the formation of new tubers, rhizomes, stolons and the like.

It has now been found that certain N-pyrazolylalkyl N-(5-pyrimidinyl)-chloroacetamides do suppress the formation of new tubers, rhizomes, stolons and the like and are therefore particularly useful in combatting perennial weeds.

The invention accordingly provides a method of controlling perennial weeds which comprise applying to the weeds or the locus thereof a regrowth suppressing amount of a compound of formula I

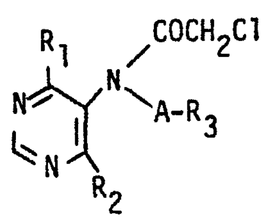

I

wherein $R_1$ is a N,N-disubstituted amino group,
$R_2$ is halogen, $C_{1-4}$alkyl, or an ether or thio ether group,
$R_3$ is 1-pyrazolyl or 3,5-dimethyl-1-pyrazolyl,
and A is $CH_2$ or $CH(CH_3)$,
in free base form or in agriculturally acceptable acid addition salt form.

Examples of perennial weeds that may be combatted with the compounds of formula I (in free base form or agriculturally acceptable salt form) are Agropyron repens, Cynodon dactylon, Cyperus esculentus, Cyperus rotundus and Sorghum halepense as established by tests on various of said perennial weeds with representative compounds of formula I with test dosages equivalent to an application rate of from 3 to 24 kg active ingredient/ha, e.g. a selection of test dosages equivalent to a rate of 3 kg, 6 kg and 12 kg or 8 kg, 16 kg and 24 kg/ha.

The compounds of formula I may be applied pre-emergence or post-emergence to the weeds. In general post-emergence treatment will require rather higher application rates than pre-emergence treatment; on the other hand post-emergence application will allow a more rational treatment.

Compounds of formula I particularly suitable for use in the method of the invention have one or more of the following features:

$R_1$ is N,N-di($C_{1-4}$alkyl)amino such as $N(CH_3)_2$, $N(C_2H_5)_2$, $N(CH_3)n-C_4H_9$ or 1-pyrrolidinyl,

$R_2$ is Cl; O-($C_{1-8}$alkyl) such as methoxy, ethoxy, propoxy (n- or iso-), butoxy (n-, iso-, sec- or tert.), pentoxy (e.g. n-pentoxy, 2,2-dimethyl-propoxy or 2-pentoxy), hexyloxy (e.g. n-hexyloxy), heptyloxy (e.g. 2-ethyl-2-methyl-butoxy); O-$C_{3-5}$alkenyl or O-$C_{3-5}$alkinyl such as $O-CH_2-CH=CH_2$, $O-CH_2-C(CH_3)=CH_2$ or $O-CH_2-C\equiv CH$; or S-$C_{1-8}$alkyl such as $S-CH_3$;

A is $CH_2$.

$R_1$ is preferably $N(CH_3)_2$, $N(C_2H_5)_2$ or 1-pyrrolidinyl, particularly $N(CH_3)_2$.
$R_2$ is preferably Cl, O-($C_{1-4}$alkyl) or S-($C_{1-4}$alkyl).
Other preferred significances of $R_2$ are $O-CH_2-CH=CH_2$, $O-CH_2-C(CH_3)=CH_2$ or $O-CH_2-C\equiv CH$.

Examples of suitable agriculturally acceptable acid addition salt forms of compounds of formula I are e.g. a hydrochloride or a hydrobromide.

The amount to be applied to attain the desired suppression of the formation of new tubers, rhizomes, stolons and the like, respectively, the desired suppression of the regrowth of perennial weeds cut before or shortly after treatment will vary depending on the target, the compound employed, mode of application, conditions and time of treatment and the like. The appropriate application rates can be determined by routine procedures by those skilled in the art, or by comparing the activity of the compounds of the invention with standards for which the application rate is known, e.g. in greenhouse tests. However, in general, satisfactory results are usually obtained when the compound is applied at a rate in the range of from about 1 to 18 kg/ha, preferably from about 3 to 12 kg/ha, more preferably from about 3 to 8 kg/ha.

The invention also provides novel compounds of formula Ia

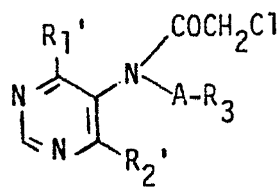

Ia

wherein $R_1'$ is $(C_{1-2}alkyl)_2$ amino or 1-pyrrolidinyl,
$R_2'$ is Cl, $C_{1-8}$alkoxy, $C_{3-5}$alkenyloxy, $C_{3-5}$alkinyloxy or $C_{1-8}$alkylthio
and A and $R_3$ are as defined above,
provided that, where $R_3$ is 1-pyrazolyl and A is $CH_2$,
then either $R_2'$ is only methoxy if $R_1'$ is 1-pyrrolidinyl
or $R_2'$ is only ethoxy if $R_1'$ is $N(CH_3)_2$ or 1-pyrrolidinyl;
or $R_2'$ is only methylthio if $R_1'$ is $N(C_2H_5)_2$ or 1-pyrrolidinyl.

The invention further provides a process for producing a compound of formula Ia by N-alkylating a compound of formula II

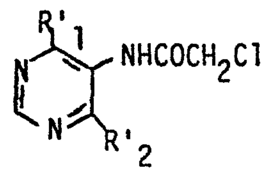

II

wherein $R'_1$ and $R'_2$ are as defined above, with a compound of formula III

$$L\text{-}AR_3 \qquad III$$

wherein L is a leaving group capable of being split off under the N-alkylation reaction conditions, and

A and $R_3$ are as defined above.

The process of the invention may be carried out by conventional manner under conditions known for the N-alkylation of amides. The reaction is advantageously carried out in a solvent which is inert under the reaction conditions e.g. dimethoxyethane, acetonitrile or methylene chloride or in an aqueous/organic two-phase system in the presence of a phase transfer catalyst such as benzyltriethylammonium chloride.

Suitable meanings of L (in formula III) are Cl, Br or the sulphonyloxy moiety of an organic sulphonic acid such as mesyloxy or p-tosyloxy.

The compounds of formula II are preferably used in salt form, more preferably in alkalimetal salt form, e.g. the sodium salt form. Such salts are obtained in conventional manner by reaction of the compound of formula II with a base such as an alkalimetal amide, hydride, hydroxide or alcoholate.

Free base forms of compounds of formula I may be converted into acid addition salt forms in conventional manner and vice versa.

The compounds of formula I e.g. the compounds of formula Ia, in free base form or in agriculturally acceptable salt form, may be and preferably are employed as herbicidal compositions in association with agriculturally acceptable diluent(s). Suitable formulations contain 0.01 % to 99 % by weight of active ingredient, 0 to 20 % agriculturally acceptable surfactant and 1 to 99.99% solid or liquid diluent(s). Higher ratios of surfactant to active ingredient are sometimes desirable and are achieved by incorporation into the formulation or by tank mixing. Application forms of composition generally contain between 0.01 and 25% by weight of active ingredient. Lower or higher levels of active ingredient can, of course, be present depending on the intended use and the physical properties of the compound. Concentrate forms of composition intended to be diluted before use generally contain between 2 and 90%, preferably between 10 and 80% by weight of active ingredient.

Useful formulations of the compounds according to the invention include wettable powders, emulsifiable concentrates and the like. They are obtained by conventional manner, e.g. by mixing the active ingredient with the diluent(s).

Agriculturally acceptable additives may be employed in the herbicidal compositions to improve the performance of the active ingredient, to improve the physical properties of the composition and the like.

Surfactant as used herein means a agriculturally acceptable material which imparts emulsifiability, spreading, wetting, dispersibility or other surface-modifying properties. Examples of surfactants are sodium lignin sulphonate and lauryl sulphate.

Diluents as used herein mean a liquid or solid agriculturally acceptable material used to dilute a concentrated material to a usuable or desirable strength. For dusts or granules it can be e.g. talc, kaolin or diatomaceous earth, for liquid concentrate forms, for example a hydrocarbon such as xylene or an alcohol such as isopropanol, and for liquid application forms i.a. water or diesel oil.

The compositions of this invention can also comprise other compounds having biological activity, e.g. compounds having similar or complementary herbicidal activity or compounds having antidotal, fungicidal or insecticidal activity.

Specific Examples of herbicidal compositions will now be described.

EXAMPLE A : Wettable Powder

25 Parts of a compound of formula I, e.g. the compound of Example 1 or of Example 2a hereinafter given, are mixed and milled with 25 parts of synthetic fine silica, 2 parts of sodium lauryl sulphate, 3 parts of sodium ligninsulphonate and 45 parts of finely divided kaolin until the mean particle size is about 5 micron. The resulting wettable powder is diluted with water before use to a spray liquor with the desired concentration.

EXAMPLE B : Emulsion Concentrate

25 Parts of a compound of formula I, e.g. the compound of Example 1 or of Example 2a hereinafter given, 50 parts of xylene, 15 parts of dimethylformamide, and 10 parts of emulsifier (e.g. ATLOX 4851 B a blend of Ca alkylarylsulphonate and a polyethoxylated triglyceride of Atlas Chemie GmbH) are thoroughly mixed until a homogeneous solution is obtained. The resulting emulsion concentrate is diluted with water before use.

NOVEL COMPOUNDS

Example 1 : N-(1-Pyrazolylmethyl)-N-(4-dimethylamino-6-ethoxypyrimidin-5-yl)-chloroacetamide

To a mixture of 12.9 g (0.05 mol) N-(4-dimethylamino-6-ethoxy-pyrimidin-5-yl)-chloroacetamide and 2 g benzyltriethylammoniumchloride in 150 g of $CH_2Cl_2$ are added 8.4 g (0.055 mol) of 1-pyrazolylmethyl-chloride-hydrochloride. To this mixture are quickly added 25 ml of 40% NaOH, whereby the temperature rises from 18° to 32°. The mixture is then stirred for 2 hours and diluted with water. The organic phase is separated off and dried over $Na_2SO_4$. The dried solution is evaporated to give an orange oil that is chromatographed on silica gel with the aid of ethylacetate. The thus obtained yellow oil is dissolved in diethylether and crystallised in a deep-freezer. The title compound is obtained in the form of white crystals of m.p. 112-114°.

Example 2

Following the procedure of Example 1 but employing the appropriate acetamide of formula II as starting material, the following compounds of formula Ia are obtained:

a) N-(1-pyrazolylmethyl)-N-(4-chloro-6-dimethylaminopyrimidin-5-yl)-chloroacetamide (m.p. 149-151°)

b) N-(1-pyrazolylmethyl)-N-[4-methoxy-6-(1-pyrrolidinyl)pyrimidin-5-yl]-chloroacetamide (m.p. 112-114°)

c) N-(1-pyrazolylmethyl)-N-[4-allyloxy-6-dimethylaminopyrimidin-5-yl-chloroacetamide (m.p. 91-94°)

d) N-(1-pyrazolylmethyl)-N-[4-dimethylamino-6-(2-methylallyloxy)pyrimidin-5-yl]-chloracetamide (m.p. 70-72°)

e) N-(1-pyrazolylmethyl)-N-[4-dimethylamino-6-(2-propinyloxy)pyrimidin-5-yl)-chloroacetamide (m.p. 116-118°)

f) N-(1-pyrazolylmethyl)-N-[4-dimethylamino-6-(n-propoxy)-pyrimidin-5-yl]-chloracetamide (m.p. 117-119°)

g) N-(1-pyrazolylmethyl)-N-[4-dimethylamino-6-(isopropoxy)-pyrimidin-5-yl]-chloracetamide (m.p. 72-75°)

h) N-(1-pyrazolylmethyl)-N-[4-dimethylamino-6-(n-butoxy)-pyrimidin-5-yl]-chloracetamide (m.p. 89-91°)

i) N-(1-pyrazolylmethyl)-N-[4-dimethylamino-6-(sec.butoxy)-pyrimidin-5-yl]-chloracetamide (m.p. 72-76°)

j) N-(1-pyrazolylmethyl)-N-[4-dimethylamino-6-(isobutoxy)-pyrimidin-5-yl]-chloracetamide (m.p. 105-106°)

k) N-(1-pyrazolylmethyl)-N-[4-dimethylamino-6-(tert.butoxy)-pyrimidin-5-yl]-chloracetamide

l) N-(1-pyrazolylmethyl)-N-[4-dimethylamino-6-(n-pentoxy)-pyrimidin-5-yl]-chloracetamide (m.p. 78-86°)

m) N-(1-pyrazolylmethyl)-N-[4-dimethylamino-6-(2-pentyloxy)-pyrimidin-5-yl]-chloracetamide (oil; mixture of isomers)

n) N-(1-pyrazolylmethyl)-N-[4-dimethylamino-6-(n-hexyloxy)-pyrimidin-5-yl]-chloracetamide (m.p. 73-75°)

o) N-(1-pyrazolylmethyl)-N-[4-dimethylamino-6-(neopentyloxy)-pyrimidin-5-yl]-chloracetamide (m.p. 108-110°)

p) N-(1-pyrazolylmethyl)-N-[4-dimethylamino-6-(2-ethyl-2-methyl-butoxy)-pyrimidin-5-yl]-chloracetamide

q) N-(1-pyrazolylmethyl)-N-[4-dimethylamino-6-(methylthio)-pyrimidin-5-yl]-chloracetamide (m.p. 113-115°)

r) N-(3,5-dimethyl-1-pyrazolylmethyl)-N-[4-dimethylamino-6-(n-butoxy)-pyrimidin-5-yl]-chloracetamide

s) N-(3,5-dimethyl-1-pyrazolylmethyl)-N-[4-dimethylamino-6-(methoxy)-pyrimidin-5-yl]-chloracetamide (m.p. 100-103°)

Use against perennial weeds

Example 3

N-(4-Dimethylamino-6-methoxy-5-pyrimidinyl)-N-pyrazolylmethyl-chloroacetamide (hereinafter compound A) is tested under greenhouse conditions (20° daylight - ca. 6000 LUX on average during 16 hours), against Cyperus rotundus, Cynodon dactylon, Agropyron repens, Sorghum halepense and Convolvulus sepium in pots. The herbicidal activity of the test compounds is evaluated after pre-em and post-em spray-application.

For pre-em evaluation the rhizomes, stolons or tubers are covered with substrate and the test substance is then applied in aqueous spray form.

For post-em evaluation the whole plant (size 20-25 cm) is treated by conventional post-em spray-application.

Beet soil is used as substrate.

The spray-application is effected under 2 conditions (600 ℓ and 2000 ℓ/ha); the results indicated in Table I are an average of 4 tests (2 application conditions, 1 replication).

The result is evaluated 14, 28, 56 and 96 days after application. After the 3 evaluation (56 days after application) the plant is cut back just above the soil level; the growth from the remaining stumps is evaluated 40 days thereafter (i.e. 96 days after application).

The results are as follows:

TABLE I - Damage in %

| Application | pre-em | | | | | | post-em | | | | | |
|---|---|---|---|---|---|---|---|---|---|---|---|---|
| Compound | Compound A | | | Standard I | | | Compound A | | | Standard I | | |
| Dosage kg a.i./ha | 3.0 | 6.0 | 12.0 | 3.0 | 6.0 | 12.0 | 3.0 | 6.0 | 12.0 | 3.0 | 6.0 | 12.0 |
| 14 DAA | | | | | | | | | | | | |
| Convolvulus | 95 | 100 | 100 | 15 | 55 | 70 | 45 | 55 | 60 | 60 | 60 | 60 |
| Agropyron | 95 | 100 | 85 | 10 | 45 | 60 | 25 | 25 | 25 | 55 | 55 | 65 |
| Cynodon dact. | 60 | 70 | 80 | 20 | 25 | 50 | 40 | 45 | 50 | 40 | 45 | 55 |
| Sorghum hal. | 35 | 35 | 40 | 45 | 55 | 60 | 20 | 30 | 35 | 45 | 45 | 50 |
| Cyperus rot. | 40 | 40 | 45 | 10 | 20 | 35 | 10 | 10 | 10 | 10 | 20 | 30 |
| 28 DAA | | | | | | | | | | | | |
| Convolvulus | 90 | 95 | 100 | 20 | 35 | 55 | 50 | 70 | 80 | 60 | 60 | 70 |
| Apropyron | 95 | 100 | 100 | 15 | 50 | 70 | 55 | 55 | 60 | 85 | 90 | 90 |
| Cynodon | 80 | 90 | 90 | 35 | 45 | 75 | 50 | 60 | 60 | 50 | 55 | 65 |
| Sorghum hal. | 60 | 75 | 75 | 20 | 60 | 65 | 50 | 60 | 65 | 60 | 65 | 70 |
| Cyperus rot. | 65 | 70 | 70 | 20 | 30 | 45 | 20 | 25 | 35 | 25 | 25 | 45 |
| 56 DAA (prior to cutting back) | | | | | | | | | | | | |
| Convolvulus | 60 | 70 | 95 | 15 | 30 | 35 | 65 | 70 | 75 | 40 | 45 | 65 |
| Agropyron rep. | 100 | 100 | 100 | 10 | 45 | 55 | 75 | 85 | 100 | 75 | 95 | 100 |
| Cynodon dact. | 75 | 85 | 95 | 20 | 40 | 65 | 70 | 75 | 85 | 50 | 60 | 70 |
| Sorghum hal. | 65 | 75 | 90 | 10 | 10 | 10 | 60 | 75 | 80 | 60 | 75 | 80 |
| Cyperus rot. | 65 | 75 | 85 | 0 | 10 | 20 | 60 | 80 | 90 | 55 | 70 | 80 |
| 96 DAA (inhibition of regrowth) | | | | | | | | | | | | |
| Convolvulus | 10 | 10 | 10 | 0 | 10 | 0 | 0 | 0 | 0 | 0 | 10 | 15 |
| Agropyron rep. | 100 | 100 | 100 | 0 | 60 | 75 | 60 | 100 | 100 | 75 | 95 | 100 |
| Cynodon dact. | 85 | 95 | 100 | 0 | 15 | 60 | 100 | 100 | 100 | 10 | 50 | 100 |
| Sorghum hal. | 75 | 75 | 95 | 0 | 50 | 75 | 20 | 30 | 35 | 20 | 35 | 60 |
| Cyperus rot. | 45 | 60 | 85 | 0 | 0 | 0 | 0 | 35 | 65 | 40 | 80 | 90 |

DAA = days after application / a.i. = active ingredient

Standard I = mixture comprising 240 g/ℓ aminotriazole + 215 g/ℓ ammoniumthiocyanate

Example 4

The herbicidal activity of compounds of Example 1, 2a, 2b and 2c hereinbefore is tested after post-em application under conditions as indicated in Example 3 hereinbefore except that the compounds are only applied at a spray-volume equivalent to 600 ℓ/ha. The results indicated in Table 2 are the average of 2 tests.

Metolachlor was used as standard.

TABLE 2 - Greenhouse - post-em trials / Damage in %

| Examples | 1 | | | 2a | | | 2b | | | 2c | | | Metolachlor | | |
|---|---|---|---|---|---|---|---|---|---|---|---|---|---|---|---|
| Dosage kg a.i./ha | 3.0 | 6.0 | 12.0 | 3.0 | 6.0 | 12.0 | 3.0 | 6.0 | 12.0 | 3.0 | 6.0 | 12.0 | 3.0 | 6.0 | 12.0 |
| 14 DAA | | | | | | | | | | | | | | | |
| Convolvulus sep. | 20 | 45 | 50 | 40 | 50 | 50 | 40 | 40 | 50 | 40 | 45 | 45 | 15 | 20 | 30 |
| Agropyron rep. | 5 | 40 | 45 | 25 | 25 | 30 | 15 | 30 | 35 | 25 | 30 | 30 | 15 | 35 | 45 |
| Cynodon dact. | 15 | 20 | 25 | 20 | 40 | 45 | 15 | 20 | 30 | 20 | 20 | 20 | 20 | 20 | 30 |
| Sorghum hal. | 0 | 0 | 0 | 0 | 0 | 0 | 0 | 0 | 10 | 10 | 10 | 15 | 0 | 0 | 30 |
| Cyperus rot. | 5 | 10 | 10 | 0 | 15 | 30 | 0 | 20 | 30 | 10 | 10 | 30 | 10 | 15 | 35 |
| 28 DAA | | | | | | | | | | | | | | | |
| Convolvulus sep. | 20 | 60 | 60 | 35 | 45 | 60 | 40 | 45 | 60 | 50 | 55 | 60 | 10 | 20 | 30 |
| Agropyron rep. | 30 | 35 | 50 | 35 | 45 | 50 | 30 | 45 | 45 | 35 | 40 | 50 | 40 | 50 | 55 |
| Cynodon dact. | 40 | 45 | 50 | 40 | 55 | 55 | 30 | 35 | 35 | 45 | 50 | 50 | 25 | 35 | 40 |
| Sorghum hal. | 0 | 20 | 25 | 0 | 0 | 15 | 0 | 20 | 25 | 20 | 20 | 25 | 0 | 0 | 0 |
| Cyperus rot. | 25 | 30 | 30 | 30 | 40 | 60 | 25 | 30 | 30 | 30 | 30 | 45 | 20 | 25 | 35 |
| 56 DAA | | | | | | | | | | | | | | | |
| (prior to cutting back) | | | | | | | | | | | | | | | |
| Convolvulus sep. | 20 | 55 | 70 | 35 | 55 | 60 | 40 | 55 | 60 | 40 | 55 | 70 | 20 | 40 | 45 |
| Agropyron rep. | 60 | 75 | 85 | 80 | 90 | 90 | 70 | 80 | 80 | 70 | 85 | 90 | 60 | 80 | 80 |
| Cynodon dact. | 60 | 60 | 70 | 60 | 70 | 70 | 45 | 60 | 60 | 60 | 65 | 70 | 50 | 50 | 50 |
| Sorghum hal. | 0 | 30 | 70 | 10 | 40 | 50 | 15 | 40 | 70 | 20 | 30 | 75 | 0 | 0 | 10 |
| Cyperus rot. | 50 | 60 | 65 | 55 | 70 | 75 | 50 | 55 | 85 | 55 | 75 | 80 | 20 | 45 | 60 |
| 96 DAA | | | | | | | | | | | | | | | |
| (% inhibition of regrowth) | | | | | | | | | | | | | | | |
| Convolvulus sep. | 0 | 40 | 60 | 0 | 50 | 70 | 0 | 30 | 30 | 0 | 0 | 25 | 0 | 0 | 0 |
| Agropyron rep | 15 | 100 | 100 | 100 | 100 | 100 | 75 | 100 | 100 | 100 | 100 | 100 | 50 | 85 | 100 |
| Cynodon dact. | 50 | 100 | 100 | 100 | 100 | 100 | 100 | 100 | 100 | 100 | 100 | 100 | 45 | 60 | 100 |
| Sorghum hal. | 50 | 90 | 100 | 20 | 70 | 70 | 20 | 80 | 100 | 20 | 70 | 100 | 0 | 0 | 35 |
| Cyperus rot. | 60 | 100 | 100 | 50 | 100 | 100 | 0 | 95 | 100 | 90 | 100 | 100 | 0 | 30 | 30 |

Example 5 : Greenhouse test (post-em)

Cyperus esculentus plants (ca. 25 cm) obtained from single tubers grown in seed pots filled with beet soil are treated with a spray solution of a compound of formula I at a rate equivalent to an application rate of 8 kg, 16 kg and 24 kg/ha (spray solume equivalent to 600 ℓ/ha.)

Analogous tests are run with Cyperus rotundus (size ca. 25 cm) except that the application rates are equivalent to 16 kg and 24 kg.

The damage is determined after visual evaluation 14 days, 28 days and 56 days after application. The day of the 3rd determination (56 days after application) the plants are cut back and the inhibition of regrowth is then expressed 96 days after application. At the same day (96 DAA) the number of new developed tubers is counted and compared with an untreated standard. It is then determined how many of those newly developed tubers are still capable of shooting.

The results are expressed in Table III in which

Compound A is N-(4-dimethylamino-6-methoxy-5-pyrimidinyl)-N-pyrazolylmethyl-chloroacetamide

B is N-[4-(N-n-butyl-N-methylamino)-6-methoxy-5-pyrimidinyl)-N-pyrazolylmethyl-chloroacetamide

C is N-(4-diethylamino-6-methoxy-5-pyrimidinyl)-N-pyrazolylmethyl-chloroacetamide

a.i. is active ingredient.

TABLE III - Greenhouse - post-em

| Test substance | Cyperus esculentus | | | | | | | | | | | | Cyperus rotundus | | | | | |
|---|---|---|---|---|---|---|---|---|---|---|---|---|---|---|---|---|---|---|
| | Compound A | | | Compound B | | | Compound C | | | Metolachlor (Standard) | | | Compound A | | Compound B | | Compound C | |
| kg a.i./h | 8 | 16 | 24 | 8 | 16 | 24 | 8 | 16 | 24 | 8 | 16 | 24 | 16 | 24 | 16 | 24 | 16 | 24 |
| % damage | | | | | | | | | | | | | | | | | | |
| 14 DAA | 10 | 10 | 10 | 0 | 17 | 10 | 7 | 10 | 17 | 0 | 10 | 20 | 0 | 20 | 0 | 0 | 0 | 0 |
| 88 DAA | 37 | 40 | 43 | 30 | 30 | 37 | 30 | 33 | 40 | 30 | 30 | 30 | 50 | 70 | 40 | 40 | 40 | 50 |
| 56 DAA(1) | 87 | 90 | 90 | 73 | 80 | 92 | 80 | 87 | 90 | 0 | 0 | 0 | 70 | 90 | 70 | 70 | 70 | 80 |
| 96 DAA(2) | 100 | 100 | 100 | 50 | 100 | 100 | 100 | 100 | 100 | 0 | 0 | 0 | 100 | 100 | 70 | 100 | 90 | 100 |
| Number of newly generated tubers(3) | 0 | 0 | 0 | 0 | 0 | 0 | 0 | 0 | 0 | 72 | 48 | 24 | 17 | 17 | 26 | 11 | 28 | 22 |
| Number of shooting newly generated tubers | - | - | - | - | - | - | - | - | - | - | - | - | 0 | 0 | 4 | 0 | 2 | 0 |

(1) prior to cutting back

(2) inhibition of regrowth in % of non-treated control

(3) in the non-treated control the number was 125 (cyperus esculentus) and 85 (cyperus rotundus; 30 thereof shooting)

- means not evaluated

What is claimed is:

1. A method of controlling perennial weeds, which comprises applying to the weeds or the locus thereof a regrowth suppresing amount of a compound of formula I

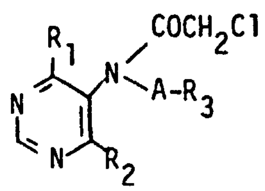

I

wherein $R_1$ is a N,N-disubstituted amino group,
$R_2$ is halogen, $C_{1-4}$alkyl, or an ether or thio ether group,
$R_3$ is 1-pyrazolyl or 3,5-dimethyl-1-pyrazolyl,
and A is $CH_2$ or $CH(CH_3)$,
in free base form or in agriculturally acceptable acid addition salt form.

2. The method of Claim 1, wherein $R_1$ is ($C_{1-4}$alkyl)amino or 1-pyrrolidinyl , $R_2$ is Cl, O-$C_{1-8}$alkyl, O-$C_{3-5}$alkenyl, O- $C_{3-5}$alkinyl or S-$C_{1-8}$alkyl.

3. The method of Claim 2, wherein $R_1$ is $(C_{1-2}alkyl)_2$amino or 1-pyrrolidinyl, $R_2$ is Cl, O-$C_{1-8}$alkyl, O-$C_{3-5}$alkenyl, O-$C_{3-5}$alkinyl or S-$C_{1-8}$alkyl, $R_3$ is 1-pyralolyl or 3,5-dimethyl-1-pyrazolyl and A is $CH_2$ or $CH(CH_3)$ provided, that where $R_3$ is 1-pyrazolyl and A is $CH_2$, then either $R_2$ is only methoxy if $R_1$ is 1-pyrrolidinyl,or $R_2$ is only ethoxy if $R_1$ is $N(CH_3)_2$ or 1-pyrrolidinyl,or $R_2$ is only methylthio if $R_1$ is $N(C_2H_5)_2$ or 1-pyrrolidinyl.

4. The method of Claim 2 or 3 wherein $R_2$ is Cl.

5. The method of Claim 2 wherein the compound of formula I is N-(4-dimethylamino-6-methoxy-5-pyrimidinyl)-N-pyrazolylmethyl-chloroacetamide.

6. The method of Claim 4 wherein the compound of formula I is N-(4-chloro-6-dimethylaminopyrimidin-5-yl)-chloroacetamide.

7. An agricultural composition for use against perennial weeds, comprising a compound of formula I, as defined in any one of Claims 1 to 6 and an agriculturally acceptable diluent.

8. A herbicidal composition comprising a compound of formula I as stated in Claim 3, 4 or 5 and an agriculturally acceptable diluent.

9. A compound of formula I as stated in Claims 3, 4 or 5.

10. A process of manufacturing compounds of formula I as stated in Claim 3, which comprises N-alkylating a compound of formula II

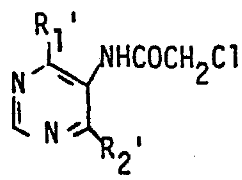

II

wherein $R_1$' and $R_2$' are as defined in Claim 3,
with a compound of formula III

$$L\text{-}AR_3 \qquad III$$

wherein L is a leaving group capable of being split off under the N-alkylation reaction conditions, and
A and $R_3$ are as defined in Claim 1.

0192606

Austria 130-3978

What is claimed is:

1. A method of controlling perennial weeds, which comprises applying to the weeds or the locus thereof a regrowth suppresing amount of a compound of formula I

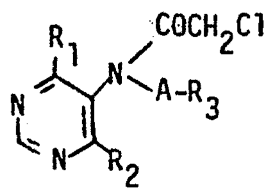

I

wherein $R_1$ is a N,N-disubstituted amino group,
$R_2$ is halogen, $C_{1-4}$alkyl, or an ether or thio ether group,
$R_3$ is 1-pyrazolyl or 3,5-dimethyl-1-pyrazolyl,
and A is $CH_2$ or $CH(CH_3)$,
in free base form or in agriculturally acceptable acid addition salt form.

2. The method of Claim 1, wherein $R_1$ is ($C_{1-4}$alkyl)amino or 1-pyrrolidinyl , $R_2$ is Cl, O-$C_{1-8}$alkyl, O-$C_{3-5}$alkenyl, O- $C_{3-5}$alkinyl or S-$C_{1-8}$alkyl.

3. The method of Claim 2, wherein $R_1$ is $(C_{1-2}alkyl)_2$amino or 1-pyrrolidinyl, $R_2$ is Cl, O-$C_{1-8}$alkyl, O-$C_{3-5}$alkenyl, O-$C_{3-5}$alkinyl or S-$C_{1-8}$alkyl, $R_3$ is 1-pyralolyl or 3,5-dimethyl-1-pyrazolyl and A is $CH_2$ or $CH(CH_3)$ provided, that where $R_3$ is 1-pyrazolyl and A is $CH_2$, then either $R_2$ is only methoxy if $R_1$ is 1-pyrrolidinyl,or $R_2$ is only ethoxy if $R_1$ is $N(CH_3)_2$ or 1-pyrrolidinyl,or $R_2$ is only methylthio if $R_1$ is $N(C_2H_5)_2$ or 1-pyrrolidinyl.

4. The method of Claim 2 or 3 wherein $R_2$ is Cl.

5. The method of Claim 2 wherein the compound of formula I is N-(4-dimethylamino-6-methoxy-5-pyrimidinyl)-N-pyrazolylmethyl-chloroacetamide.

6. The method of Claim 4 wherein the compound of formula I is N-(4-chloro-6-dimethylaminopyrimidin-5-yl)-chloroacetamide.

7. An agricultural composition for use against perennial weeds, comprising a compound of formula I, as defined in any one of Claims 1 to 6 and an agriculturally acceptable diluent.

0192606

Austria

130-3978

8. A herbicidal composition comprising a compound of formula I as stated in Claim 3, 4 or 5 and an agriculturally acceptable diluent.

9. A process of manufacturing compounds of formula I as stated in Claim 3, which comprises N-alkylating a compound of formula II

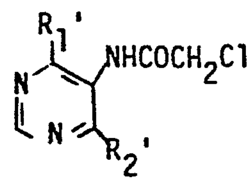

II

wherein $R_1$' and $R_2$' are as defined in Claim 3, with a compound of formula III

$$L\text{-}AR_3 \qquad III$$

wherein L is a leaving group capable of being split off under the N-alkylation reaction conditions, and

A and $R_3$ are as defined in Claim 1.